# FASCICULE DE BREVET EUROPEEN

(11) **EP 2 825 213 B1**
(45) Date de publication et mention de la délivrance du brevet: **13.04.2016**
(21) Numéro de dépôt: 13715281.5
(22) Date de dépôt: 08.03.2013
(51) Int. Cl.: A61L 2/00, A61L 2/04, B65B 55/14, B65B 55/18, A23L 3/10, B01L 7/02, B01J 3/04

(54) **DISPOSITIF DE TRAITEMENT THERMIQUE APTE A RECEVOIR DES PRODUITS EN VRAC**
WÄRMEBEHANDLUNGSVORRICHTUNG ZUR AUFNAHME VON SCHÜTTGUT
HEAT-TREATMENT DEVICE CAPABLE OF RECEIVING LOOSE PRODUCTS

(30) Priorité: 16.03.2012 FR 1252401
(43) Date de publication de la demande: 21.01.2015
(73) Titulaire: Lagarde, 26780 Malataverne (FR)
(72) Inventeur: RANCHON, Denis, F-07220 Vivier (FR)
(74) Mandataire: Domange, Maxime
(86) Numéro de dépôt international: PCT/FR2013/050488
(87) Numéro de publication internationale: WO 2013/135998

(56) Documents cités:
- CH-A- 406 810
- FR-A- 1 036 490
- FR-A- 1 564 529
- FR-A1- 2 290 249
- FR-A1- 2 808 706

## Description

### Arrière-plan de l'invention

La présente invention se rapporte au domaine général des dispositifs de traitement thermique de produits sous emballage disponibles en vrac comprenant une enceinte destinée à être mise sous pression et à mettre en oeuvre un tel traitement thermique par immersion des dits emballages dans de l'eau chauffée à température donnée , notamment des traitement de stérilisation, notamment à T=100 à 125°C ou pasteurisation, notamment à T= 80-90°C. La mise sous pression vise à maintenir l'eau à l'état liquide à la dite température le cas échéant et vise aussi à conférer une contre pression qui maintient en forme les emballages qui du fait du chauffage se déforme du fait de l'augmentation de pression et volume du gaz contenu dans l'emballage le cas échéant notamment pour des emballages souples en matière plastique.

Actuellement, pour automatiser une chaine de stérilisation en amont et en aval d'une enceinte de traitement thermique, aussi appelé autoclave, il est nécessaire d'avoir des accessoires spécifiques tels que paniers, claies, plateaux destinés à porter des produits stérilisés sur des chaînes de production et/ou dans les autoclaves et un système de convoyage, chargement, déchargement. Les autoclaves sont conçus pour accueillir les accessoires, paniers, claies, plateaux, dans lesquels sont stockés les produits. FR 2 290 249 décrit un tel dispositif dans lequel des produits emballés sont placés dans des caissettes avant d'être introduits dans l'enceinte.

Un tel système est couteux financièrement. Il est aussi couteux en termes d'espace et encombrement dans les usines puisque le système de chargement/déchargement doit trouver devant ou derrière l'autoclave. Selon le nombre d'autoclaves, et donc de jeu d'accessoires, un espace de stockage des accessoires doit également être prévu.

Par ailleurs, comme il est nécessaire de remplir les dits accessoires avant l'acheminement de ceux-ci vers et/ou dans l'autoclave puis de les vider après la stérilisation, les procédés connus sont très couteux en termes de temps de production.

En outre une très grande attention est requise d'un point de vue maintenance et entretien pour éviter les défaillances mécaniques. Peu de solutions existent à ce jour en matière d'automatisation complète de la chaîne de traitement.

Concernant la gestion des produits traités, les procédés connus ne permettent pas de détecter visuellement et directement un mauvais scellage, la porosité et/ou la fuite d'un emballage. FR 1 036 490 vise à traiter de manière automatisé des produits vrac. Cependant, les produits traités sont dits « coulants » et le dispositif ne comprend pas de bac, mais est constitué d'un tube en serpentin.

### Objet et résumé de l'invention

La présente invention a donc pour but principal de pallier les inconvénients des procédés et autoclaves connus et de permettre un traitement thermique simplifié des produits en proposant un dispositif de traitement thermique de produits sous emballage disponibles en vrac comprenant une enceinte destinée à être mise sous pression et à mettre en oeuvre un tel traitement thermique, caractérisé en ce que le dispositif comprend :
- un bac destiné à contenir un fluide d'une densité telle que les produits (P) sous emballage flottent lorsqu'ils y sont déversés en vrac, ce bac présentant des moyens d'évacuation des produits en état de flottaison, ces moyens d'évacuation comprenant une trappe apte à être immergée puis ouverte pour permettre l'évacuation d'une partie du fluide comportant les produits (P) en état de flottaison,
- des moyens de fourniture de fluide pour remplir le bac et réguler le niveau de fluide dans le bac
- des moyens d'immersion (aussi dénommés ci-après « presseurs ») agissant au niveau de la surface supérieure de fluide présent dans le bac pour immerger les produits en vrac dans le fluide,
- des moyens de récupération des produits au sein du fluide évacué du bac lors de l'ouverture du compartiment d'évacuation,
- des moyens de réception du fluide déversé lors de l'ouverture du compartiment d'évacuation.

Plus particulièrement, la dite trappe est située au niveau d'une paroi latérale notamment la paroi avale ou avant en partie supérieure de la paroi du bac.

Un tel dispositif de traitement thermique permet un chargement par acheminement direct dans le bac, typiquement par convoyage simple des produits, permettant de s'abroger entièrement desdits accessoires ainsi que de tous systèmes de navette, de quais d'amenée etc.

Au déchargement, il est aussi possible de mettre en place un acheminement direct des produits vers l'extérieur sans accessoires, ni système de navette ou de quai de sortie comme cela est le cas aujourd'hui.

Des économies substantielles en termes d'investissement en infrastructures sont réalisées. En outre le nouveau dispositif permet de gagner de la place puisqu'il n'est plus utile de prévoir des zones pour stocker et pour manipuler les accessoires.

Au niveau du temps de productivité, les doubles opérations de remplissage d'accessoires sont économisées puisque les produits sont chargés directement dans le bac de l'autoclave.

Des principes simples de déplacement des produits par circulation du fluide dans le bac sont utilisés au sein du dispositif selon l'invention ce qui réduit au maximum les systèmes mécaniques.

En outre, parmi les avantages additionnels, le dispositif permet que les emballages défectueux non étanches coulent au fond du bac. Cela permet leur visualisation et leur élimination par non évacuation lors du déchargement des produits par flottaison. Cela permet d'améliorer le contrôle sur la sécurité des aliments.

Dans un mode préféré de réalisation, le dit bac est amovible pouvant être introduit et sortie de la dite enceinte par au moins une porte de la dite enceinte, de préférence ledit bac étant aptes à coopérer avec des moyens de déplacement tels que des rails et/ou glissières sur lesquels il est posé.

On remarque aussi que l'invention nécessite ainsi des modifications mineures au sein des enceintes autoclaves connues à ce jour puisque le bac peut être installé sur les rails et autres dispositifs permettant l'amenée des accessoires. Les aménagements nécessaires à la mise en place du bac et mise en oeuvre de l'invention sont donc mineurs et peuvent être réalisés à bas cout puisque l'enceinte reste une enceinte standard. La transformation de l'enceinte peut aussi être aisément réalisée de manière réversible de manière à pouvoir revenir à un dispositif permettant de traiter des produits non flottants par des traitements conventionnels.

On note enfin que l'enceinte subit moins de sollicitations mécaniques puisque les chocs thermiques ne sont pas directement appliqués sur le corps de l'enceinte mais sur le bac lequel est plus facilement réparable et/ou remplaçable ceci d'autant plus s'il est amovible et coopère avec des moyens de déplacement tels que des rails sur lesquels il est posé. La durée de vie de la partie chaudronnée de l'enceinte est donc allongée.

Selon une caractéristique avantageuse, le dit bac comporte, de préférence à une extrémité en vis-à-vis d'une porte de l'enceinte, un compartiment supérieur dénommé compartiment d'évacuation dont une paroi forme ou comprend une dite trappe d'évacuation du compartiment d'évacuation, la dite trappe étant ouvrable, de préférence par pivotement ou coulissement, le dit compartiment d'évacuation étant disposé de telle sorte que le nombre de produit à l'intérieur dudit compartiment d'évacuation en vis-à-vis de la dite trappe soit en nombre inférieur au nombre de produit total contenu dans le bac.

Ce compartiment permet ainsi de mieux contrôler l'évacuation des produits par la dite trappe en limitant le nombre de produits en regard de la trappe.

Plus particulièrement, le dit compartiment d'évacuation constitue une structure en surplomb s'étendant sur un des côtés du bac. La trappe du compartiment d'évacuation peut ainsi être placée à une extrémité longitudinale du bac orientée vers une sortie de l'enceinte ce qui autorise une gestion plus facile de l'évacuation des produits traités.

Selon une caractéristique avantageuse, le dit compartiment supérieur comprend un plancher s'étendant horizontalement au-dessus du plancher de l'enceinte, et s'étendant au-delà et à partir du sommet d'une paroi transversale verticale inférieure du bac jusque vers la porte de sortie de l'enceinte, ladite paroi transversale verticale inférieure du bac délimitant une partie inférieure du bac dessous et amont du dit compartiment d'évacuation. Ainsi, du fait de la diminution de la hauteur d'eau au niveau du compartiment d'évacuation, le flux de circulation du fluide et des produits évacués au niveau de la trappe lorsqu'elle est ouverte est accélérée ce qui permet de mieux régulé l'évacuation des produits par la dite trappe.

D'autre part, ceci permet de distinguer une partie inférieure du bac en amont dudit compartiment d'évacuation plus profonde que le dit compartiment, au sein de laquelle on réalise l'immersion totale des produits à l'aide de dits presseurs et sur la paroi de fonds de laquelle on peut récupérer les produits non flottants dont l'emballage est endommagé.

Plus particulièrement encore, la dite trappe du compartiment d'évacuation est placée au niveau d'une paroi transversale verticale dudit compartiment d'évacuation à une extrémité du bac disposée en vis-à-vis d'une sortie de l'enceinte.

On comprend que cette paroi latérale transversale d'extrémité dudit compartiment d'évacuation constitue la partie supérieure de la paroi latérale transversale du bac à cette extrémité.

La structure en surplomb du compartiment d'évacuation par rapport au plancher de l'enceinte à l'extérieur sur un des côtés du bac, permet aussi de libérer l'espace sous une partie du bac pour y disposer un récipient de réception du fluide séparé des produits en cours d'évacuation sur ladite rampe ou goulotte perforée .

Avantageusement, les moyens de récupération des produits au sein du fluide évacué du bac lors de l'ouverture de la dite trappe comprennent une rampe ou goulotte inclinée, de préférence perforée, s'étendant à l'extérieur du dit bac depuis juste dessous la dite trappe et sont associés à des moyens de déshumidification ou de séchage des produits évacues sur la dite rampe ou goulotte. Cette caractéristique permet de réduire encore les durées de traitement en combinant deux étapes du procédé de traitement des produits.

Selon une variante de réalisation, la trappe du compartiment d'évacuation est placée sous la structure en surplomb.

Préférentiellement, la trappe du compartiment d'évacuation est placée en extrémité de la structure en surplomb. Avec cette caractéristique, on permet une régulation plus aisée et un flux de sortie des produits plus contrôlé.

Selon une caractéristique particulière de l'invention, les moyens mécaniques d'immersion comprennent une ou plusieurs plaques percées ou une ou des grilles aptes à être montées et descendues entre une position émergée et une position immergée, position immergée dans laquelle les produits, piégés sous la plaque ou la grille, peuvent être immergés, de préférence dessous la dite trappe.

Cette réalisation des moyens d'immersion est particulièrement simple et efficace. Elle permet d'immerger mécaniquement les produits de manière sure et simple.

Selon une caractéristique avantageuse, les moyens de fourniture de fluide comprennent des entrées de fluide tels que notamment des buses de refoulement, situés du côté opposé au compartiment d'évacuation, de préférence sur une dite paroi latérale transversale verticale amont du bac de préférence encore au-dessus et au-dessous du plancher du dit compartiment d'évacuation, de préférence encore à différentes hauteur au-dessus et au-dessous du plancher du dit compartiment d'évacuation, et sont aptes à injecter du fluide pour diriger les produits flottants en direction du compartiment d'évacuation d'évacuation. Cette caractéristique permet d'activer la circulation des produits en flottaison lors de leur évacuation.

Selon une caractéristique avantageuse, le dispositif comprend des moyens de régulation de l'ouverture de la trappe en fonction du niveau d'eau dans le bac et le compartiment et/ou de la vitesse d'évacuation des produits. Une telle régulation permet ainsi d'adapter la rapidité d'évacuation des produits en fonction de leur nombre, de leur taille ou encore de l'amenée de fluide.

Avantageusement, ledit bac comprend des moyens formant une deuxième paroi de fond ouvrable disposée au-dessus de la première paroi de fond, la dite deuxième paroi de fond étant apte à adopter :
- une position de fermeture empêchant que des produits coulés ou des parties de produits coulés d'un lot précédent reposant sur la première paroi de fond ne se mélangent avec les produits flottants d'un lot suivant, à traiter thermiquement, immergés dans le fluide au-dessus de la deuxième paroi de fond, les produits non flottants dudit lot suivant étant déposés sur ladite deuxième paroi de fond en position de fermeture, et
- une position d'ouverture permettant de laisser tomber sur la dite première paroi de fond, les produits non flottants reposant précédemment sur ladite deuxième paroi de fond.

En dite position de fermeture les première et deuxième parois de fond dudit bac délimitent un compartiment aptes à piéger les produits non flottants.

Plus particulièrement, lesdits moyens formant la deuxième paroi de fond comprennent deux volets pivotants montés articulés sur les parois latérales longitudinales opposées dudit bac au-dessus de ladite première paroi de fond, celle-ci comprenant une deuxième trappe d'évacuation, les dits volets étant aptes à flotter de sorte que :
- lorsque le niveau de fluide est situé au-dessous des bords des volets équipés de charnières fixées sur les dites parois longitudinales, les dits volets sont en dite position d'ouverture, et
- lorsque le niveau de fluide est situé au-dessus des bords des volets équipés de charnières fixées sur les dites parois longitudinales, les dits volets sont en dite position de fermeture.

L'invention concerne également un procédé de traitement thermique de produits sous emballage disponibles en vrac mettant en oeuvre une enceinte destinée à être mise sous pression, de préférence avec un dispositif selon l'invention tel que défini ci-dessus, caractérisé en ce que le procédé comprend les étapes suivantes consistant à:
- remplir un bac placé dans une enceinte avec un fluide
- remplir le bac avec un lot de produits P sous emballage en vrac , ledit fluide étant d'une densité telle que les produits sous emballage flottent lorsqu'ils y sont déversés dans le dit bac , le bac présentant des moyens d'évacuation des produits en état de flottaison comprenant une trappe apte à être immergée,
- immerger les produits mécaniquement,
- clore l'enceinte,
- procéder au traitement thermique au sein de l'enceinte par chauffage de l'eau au sein dudit bac et mise en pression de l'intérieur de l'enceinte,
- laisser remonter en flottaison les dits produits,
- ouvrir la trappe d'évacuation des produits en état de flottaison,
- alimenter le dit bac en fluide et faire circuler le fluide et les produits pour réguler l'évacuation du fluide et des produits (P) au niveau de la trappe,
- récupérer lesdits produits au sein du fluide évacué à l'extérieur du bac,
- récupérer le fluide déversé.

Plus particulièrement, le procédé comprend les étapes suivantes consistant à :
- remplir de fluide un dit bac présentant un dit compartiment d'évacuation des produits en état de flottaison comprenant une trappe apte à être immergée, jusqu'à un premier niveau situé dessous la dite trappe,
- déverser les dits produits en vrac au sein dudit bac,
- immerger les dits produits mécaniquement,
- clore ladite enceinte,
- procéder au traitement thermique au sein de ladite enceinte,
- ajouter dudit fluide au sein dudit bac jusqu'à un second niveau permettant l'immersion dudit compartiment d'évacuation et de ladite trappe,
- laisser remonter en flottaison les dits produits,
- ouvrir ladite trappe dudit compartiment d'évacuation desdits produits en état de flottaison,
- alimenter le dit bac en dit fluide et faire circuler le fluide et les produits pour réguler l'évacuation du fluide et des dits produits au niveau de la trappe, en régulant le niveau de fluide au sein dudit bac pendant l'évacuation des dits produits,
- récupérer les dits produits au sein du fluide évacué dudit bac,
- récupérer le fluide déversé.

Dans un mode de réalisation du procédé, on réalise les étapes suivantes dans lesquelles :
- les produits non flottants sont déposés sur des moyens formant une deuxième paroi de fond en position de fermeture disposée au-dessus de la première paroi de fond, la dite deuxième paroi de fond étant apte à adopter:
- une position de fermeture empêchant que des produits coulés ou des parties de produits coulés d'un lot précédent reposant sur la première paroi de fond ne se mélangent avec les produits flottants d'un lot suivant, à traiter thermiquement, immergés dans le fluide au-dessus de la deuxième paroi de fond, les produits non flottants dudit lot suivant étant déposés sur ladite deuxième paroi de fond en position de fermeture, et
- une position d'ouverture permettant de laisser tomber sur la dite première paroi de fond, les produits non flottants reposant précédemment sur ladite deuxième paroi de fond, et
- après que tous les produits flottants aient été évacués par la dite trappe d'évacuation, on ouvre la dite deuxième paroi de fond pour déposer sur la dite première paroi de fond , les produits non flottants reposant précédemment sur ladite deuxième paroi de fond.

Avantageusement, après plusieurs cycles de traitement thermique, si nécessaire, on sort le bac amovible de l'enceinte pour en assurer la maintenance ou le remplacement.

Un avantage essentiel des dispositif et procédé selon l'invention réside en effet dans
la mise en oeuvre d'un bac amovible que l'on peut retirer le bac ce qui permet de :
- pouvoir le remplacer et/ou en assurer la maintenance plus facilement que les parois de l'enceinte si l'enceinte faisait office de bac, et
- mettre en oeuvre l'enceinte pour réaliser un autre type de traitement thermique par autoclavage par vapeur sous pression par exemple en retirant le dit bac.

La facilité de remplacement ou manutention du bac amovible est particulièrement avantageuse du fait que c'est celui-ci qui subit des contraintes mécaniques du fait de la pression à l'intérieur de l'enceinte et la température de l'eau à l'intérieur du bac, et non les parois de l'enceinte principale.

### Brève description des dessins

D'autres caractéristiques et avantages de la présente invention ressortiront de la description faite ci-dessous, en référence aux dessins annexés qui en illustrent un exemple de réalisation dépourvu de tout caractère limitatif. Sur les figures :
la figure 1 montre un dispositif selon l'invention ;
la figure 2 montre schématiquement l'étape de déversement des produits du procédé de traitement thermique dans un dispositif selon l'invention ;
la figure 3 montre schématiquement l'étape d'immersion des produits dans un procédé de traitement thermique selon l'invention ;
la figure 4 montre schématiquement l'étape de dé-immersion des produits à l'issue du traitement thermique dans un dispositif selon l'invention ;
la figure 5 montre schématiquement l'étape de poussée des produits vers le compartiment d'évacuation dans un dispositif selon l'invention ;
la figure 6 montre schématiquement l'étape d'évacuation des produits dans un procédé de traitement thermique selon l'invention ;
la figure 7 montre schématiquement la fin du procédé de traitement thermique selon l'invention.
La figure 8 représente une variante de réalisation du dispositif sans l'enceinte.
Les figures 9A et 9B montrent schématiquement en coupe transversale verticale, le bac équipé de volets pivotants aptes à former une deuxième paroi de fond 7 au-dessus d'une première paroi de fond à section incurvée.

### Description détaillée d'un mode de réalisation

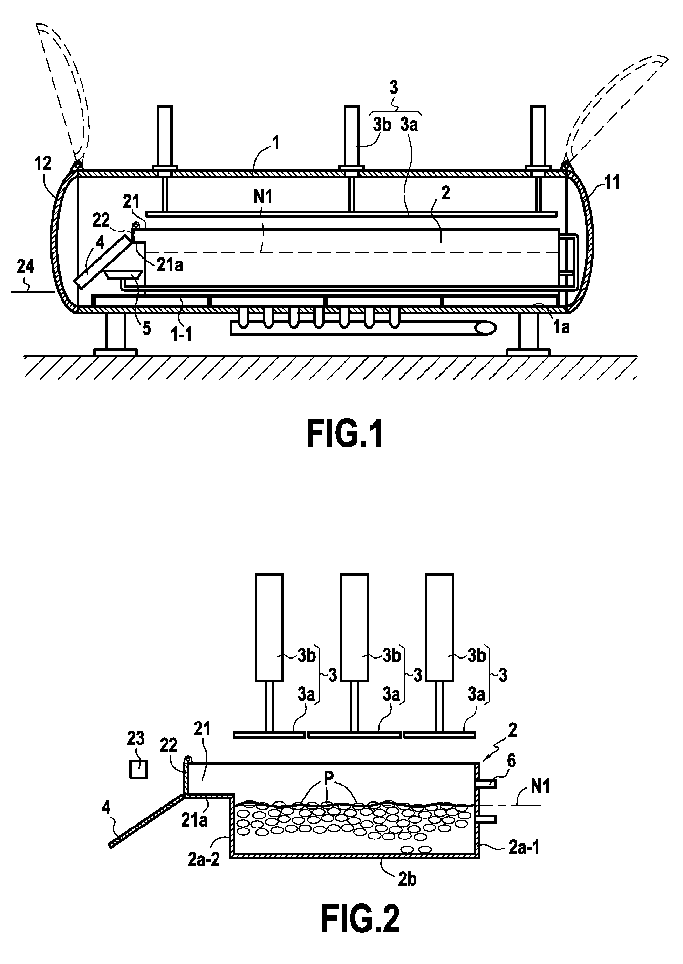

La figure 1 montre un dispositif selon l'invention. Il comprend une enceinte 1 qui est avantageusement une enceinte autoclave classique préalablement utilisée avec des accessoires. Cette enceinte 1 comprend un corps sensiblement cylindrique d'axe longitudinal horizontal comprenant à chaque extrémité longitudinale une porte d'entrée 11 et respectivement une porte de sortie 12.

L'aménagement interne est modifié selon l'invention. Pour cela, un bac à eau 2 ouvert en surface est installé de manière amovible à l'intérieur de l'enceinte 1 par les portes d'entrée 11 ou sortie 12. Ledit bac est posé et apte à être déplacé sur des rails 2-1. Le bac 2 présente une partie inférieure à parois latérales parallélépipédiques avec une paroi de fond 2b plane ou incurvée dont une paroi latérale transversale d'extrémité amont 2a-1 en vis-à-vis d'une porte d'entrée 11 à une extrémité de l'enceinte 1 et une paroi latérale transversale d'extrémité avale 2a-2 en vis-à-vis d'une porte de sortie 12 à l'autre l'extrémité de l'enceinte 1. La paroi 2a-2 est moins haute que la paroi 2a-1. La paroi 2a-2 se prolonge par une paroi horizontale 21a en porte à faux s'étendant vers la porte de sortie 12 de l'enceinte. La paroi ou plancher 21a se termine par une paroi latérale transversale formant ou comprenant une trappe pivotante 22, délimitant avec le plancher 21a et les extrémités des autres parois latérales longitudinales du bac 2 un compartiment supérieur du bac dénommé compartiment d'évacuation de sortie.

Le bac 2 comprend ainsi un compartiment d'évacuation 21 en surplomb sur un des côtés du bac 2 positionné du côté de la porte de sortie 12.

Ce compartiment d'évacuation 21 comprend une trappe 22 situé en extrémité latérale du compartiment d'évacuation 21 et donc orienté vers la porte de sortie 12. Mais, dans un mode de réalisation la trappe 22 pourra être intégrée dans le plancher 21a du compartiment d'évacuation 21. Cette trappe 22 débouche dans des moyens de récupération des produits 4, typiquement une rampe ou goulotte inclinée en pente descendante depuis le compartiment d'évacuation 21 en hauteur vers le plancher de l'enceinte à l'extérieur du bac.

La rampe ou goulotte d'évacuation des produits P est associée à des moyens de récupération du fluide déversé avec les produits P par la trappe dans la goulotte lors de l'ouverture de la trappe comprenant des perforations aptes à retenir les produits P et laisser s'écouler le fluide et des moyens de réception 5 du fluide disposé sous la goulotte tel qu'un récipient ou le fond de l'enceinte 1. Les moyens de réception du fluide 5 sont avantageusement couplés avec des moyens de recyclage qui recyclent le fluide vers le bac pour une réutilisation dans le procédé de traitement. Typiquement le fluide recyclé sera réchauffé afin d'être réinjecté lors du remplissage du bac pour un cycle de traitement ultérieur ou sera réinjecté en circuit fermé au niveau de la buse de refoulement 6 au niveau de la paroi latérale transversale amont 2a-1 pour faire circuler les produits ainsi que décrit ultérieurement.

Avant la mise en oeuvre du procédé, le bac à eau 2 est rempli jusqu'à un premier niveau N1 ou niveau bas. Ce niveau N1 est situé en dessous de la trappe 22. Ici, comme le compartiment d'évacuation est en surplomb et que la trappe ferme l'extrémité du compartiment d'évacuation 21, le niveau N1 est donc en dessous de la surface basse 21a du compartiment d'évacuation 21.

Le fluide est alors à une température constante de quelques dizaines de degrés, avantageusement préchauffé donc.

Préférentiellement, pendant cette phase, la porte d'entrée 11 est ouverte pour permettre l'alimentation du bac 2 en produit P venant de l'extérieur de l'enceinte 1 et entrant dans l'enceinte par la porte 11, et la porte de sortie 12 est fermée.

La figure 2 montre la première étape du procédé de traitement thermique selon l'invention. Les produits à traiter P sont convoyés et déversés en vrac dans le bac à eau 2. Une fois la quantité idoine d'emballages à traiter atteinte, la porte d'entrée 11 est fermée. Un bac par exemple de 5 à 15m², de préférence 7-8m² de surface de plancher 2b et de hauteur de paroi 2a de 1m-1,50m pourra contenir 5 à 10 T de produits , plus particulièrement 6-8T représentant par exemple un lot (batch) de 10000 à 15000 produits P de 100-250g disposés sur au moins 3 couches superposées dans la partie au-dessus du plancher 2b du bac 2.

Au-dessus de la partie supérieure ouverte du bac 2 sont installés des moyens mécaniques d'immersion 3. Ces moyens sont ici des plaques horizontales percées ou des grilles 3a dont les perforations sont d'une taille inférieure à la taille du plus petit produit susceptible d'être traités dans l'enceinte 1. Ces plaques d'immersion sont actionnables en déplacement vertical par des vérins 3b.

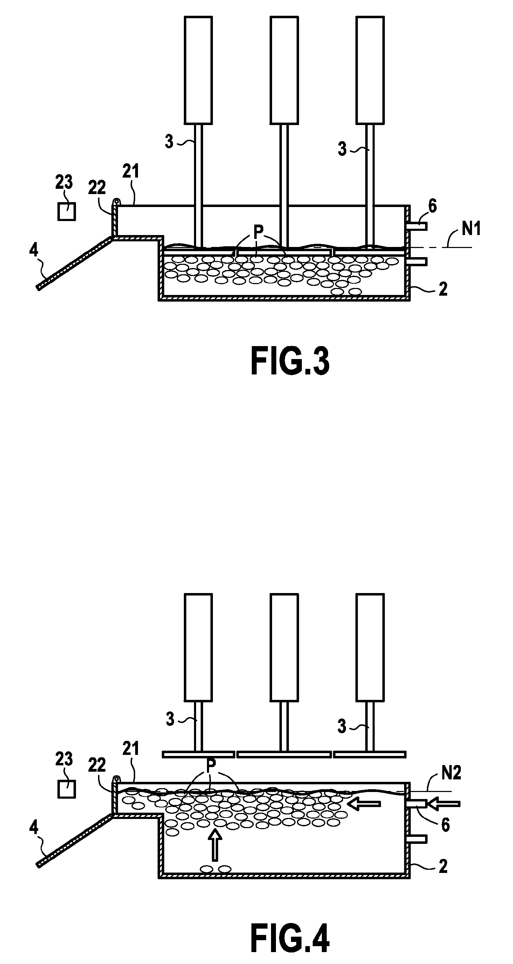

Ainsi qu'illustré sur la figure 3, les moyens d'immersion 3 sont alors actionnés en position d'immersion forcée des produits en surface. Les plaques sont descendues mécaniquement jusque sous le niveau N1 afin d'assurer l'immersion complète des produits P. on assure ainsi que l'intégralité des produits soit traitée thermiquement lorsque l'eau sera chauffée ultérieurement. Ainsi les plaques d'immersion sont descendues, par exemple à l'aide de vérins dont les tiges traversent le plafond de l'enceinte par des piquages typiquement déjà existants dans les enceintes existantes pour des amenées de vapeur ou autres.

On remarque ici que les emballages défectueux se déposent alors naturellement au fond du bac 2 en raison de leur non flottaison.

On effectue la fermeture des portes 11 et 12, puis la mise en pression de l'intérieur de l'enceinte par exemple à pression de 10⁵ à 5.10⁵.Pa (1 à 5 bars), notamment en 2.10⁵ à 3,5.10⁵.Pa (2 à 3,5 bars).

Le traitement thermique proprement dit est alors réalisé par chauffage de l'eau dans le bac jusqu'à la température prévue, cette température étant maintenue durant un temps d'immersion prédéterminé, par exemple 121°C pendant ¾ hr à 2hr pour stérilisé tout le lot de produits immergés.

Une phase de refroidissement des emballages suit jusqu'à une température souhaitée.

Une fois le cycle de traitement thermique terminé, une mise à un nouveau niveau N2 du bac 2 au-dessus du plancher 21a est alors réalisée. Cette mise à niveau peut être réalisée par les moyens d'amenée d'eau qui ont servi à remplir le bac 2. Avantageusement elle sera réalisé à l'aide d'entrée de fluide, typiquement des buses de refoulement 6 situées du côté amont opposé au compartiment d'évacuation 21 (en aval). Le niveau N2 correspond au niveau auquel le compartiment d'évacuation 21 est au moins partiellement rempli permettant le déchargement des emballages P par la trappe 22 ouverte du compartiment d'évacuation 21 selon un système de type écluse.

La figure 4 montre le bac après mise à niveau N2 lors de l'étape de remontée des plaques d'immersion 3 illustrée par des flèches. Les produits P sont alors libérés en flottaison et remontent naturellement à la surface du fluide dans le bac 2. Les premiers emballages remontés en surface sont alors dirigés vers le compartiment d'évacuation 21 par un courant d'eau généré par les buses de refoulement 6.

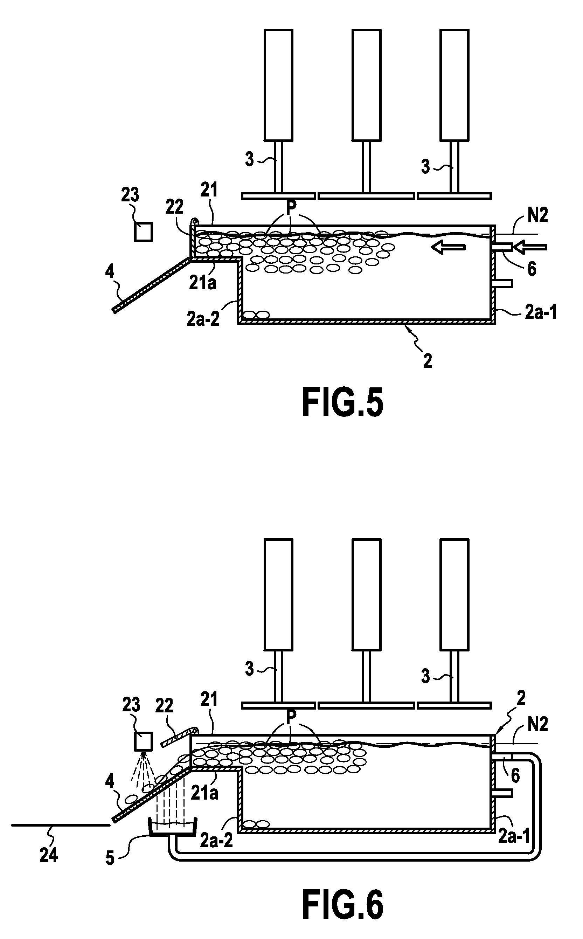

La figure 5 montre que le compartiment d'évacuation 21 en aval permet d'une part de limiter la quantité de produits en vis-à-vis de la trappe 22 à l'issue de cette étape de poussée des produits P.

Ensuite, la porte de sortie 12 peut être ouverte alors que la porte d'entrée 11 reste fermée, et la trappe 22 du compartiment d'évacuation 21 est ouverte et les premiers emballages commencent à tomber sur une goulotte 4 qui récupère les produits P dans le flux de fluide.

Les emballages suivants, grâce au principe d'Archimède, remontent alors à la surface et dans le compartiment d'évacuation 21 et sont acheminés par le courant d'eau maintenu par les buses 6 vers le compartiment d'évacuation 21. Cette étape est illustrée sur la figure 6 qui montre un autre effet technique avantageux du compartiment d'évacuation 21 lequel du fait de sa hauteur réduite par rapport à la hauteur total du bac permet de susciter une accélération du débit des produits et fluide sortant par la trappe 22.

Le fluide évacué à la sortie des emballages est récupéré ici dans un récipient 5 posé sur le fond de l'enceinte 1 ou directement dans le fonds de l'enceinte qui réceptionne le fluide dessous la goulotte 4 et avantageusement la recycle pour une réinjection dans le bac 2 par les buses de refoulement 6. Une partie de l'eau peut aussi être récupérée pour être réchauffée puis réinjectée lors d'un cycle suivant. Cela permet de conserver le niveau N2 et le courant d'eau nécessaire à l'évacuation continue des produits P.

Avantageusement, la goulotte 4 est associée avec des moyens de déshumidification 23 sur le chemin de sortie des emballages de l'enceinte 1, par exemple des moyens de soufflage d'air sur le produits P, les dits moyens 23 étant donc disposés au-dessus de la rampe 4. Ceci évite de devoir prévoir un acheminement spécifique pour la déshumidification.

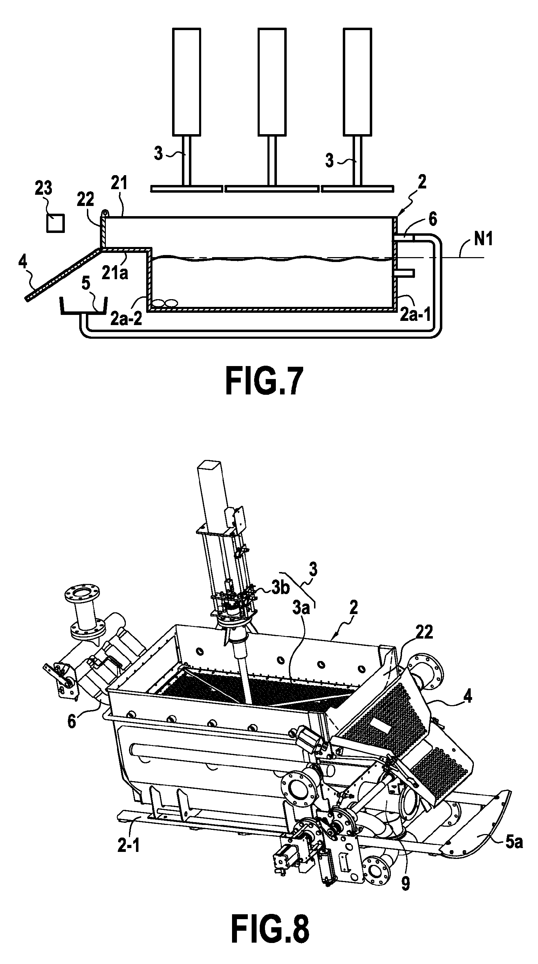

La figure 7 illustre schématiquement le bac à eau 2 à l'issue de l'évacuation des produits P après arrêt du fonctionnement des buses de refoulement 6. Tous les emballages ayant eu un problème d'étanchéité par scellage, soudage ou collage défaillant sont restés au fond 2b du bac 2. Le contrôle qualité est alors plus rapide et aisé en sortie du dispositif de traitement thermique.

Le niveau de fluide est ramené au niveau N1 initial. La trappe 22 se ferme alors et le dispositif est prêt pour accueillir un nouveau chargement de produits en vrac.

Avantageusement le fluide utilisé est de l'eau. Le dispositif selon l'invention est particulièrement adapté pour les emballages souples mais peut également être utilisé pour les barquettes et autres emballages étanches devant subir un traitement thermique et ayant une densité compatible avec une flottaison dans le fluide utilisé dans le dispositif selon l'invention.

Une trappe annexe située sur le bas du bac à eau 2 sera avantageusement installée pour pouvoir aller récupérer les emballages ayant coulé au fond du bac 2, par exemple avec un râteau ou autre outil.

On remarque enfin que diverses mises en oeuvre peuvent être réalisées selon les principes de l'invention. En particulier la trappe pourra être installée au niveau de la surface inférieure 21a du compartiment d'évacuation.

De même, le plancher 21a peut être supprimé et la trappe 22 disposé sur la partie supérieure d'une paroi latérale transversale 2a-2 surélevée. Mais ceci implique une gestion précise de son ouverture et de la régulation du remplissage du bac et débit d'évacuation des produits.

Sur la figure 8, le dispositif comporte des patins 1-1 aptes à coopérer avec des rails 2-1 déposés sur le fond la de l'enceinte. Le dispositif comporte un unique presseur 3 avec un grand plateau perforé 3a recouvrant toute la surface du bac en amont du compartiment d'évacuation (le décroché de la paroi latérale avant du bac formant le compartiment d'évacuation 21 n'est visible car il est caché par la trappe 22).D'autre part, le fluide est récupéré directement dans le fond de l'enceinte , celui-ci étant délimité par une tôle de retenue dont la forme incurvée du bord inférieur épouse la forme de la section du fond de l'enceinte. La dite tôle de retenue 5a est située en aval de l'enceinte de manière à pouvoir retenir le fluide dans le fond de l'enceinte lorsque la porte avale 11 de l'enceinte est ouverte pour permettre l'évacuation des produits sur un convoyeur 24 à l'extérieur de l'enceinte.

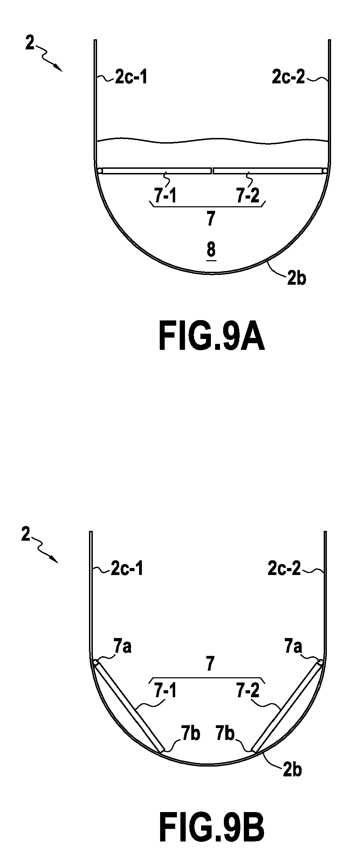

Sur les figures 9A et 9B, on a représenté un mode de réalisation dans lequel le bac 2 est équipé d'un double fond, la première paroi de fond 2b étant surmontée d'une deuxième paroi ouvrable 7 constituée par 2 volets pivotants 7-1, 7-2 montés, en vis-à-vis l'un de l'autre, sur les 2 parois latérales longitudinales du bac 2c-1,2c-2. Les dits volets sont articulés en rotation de telle sorte que les bords 7b des deux volets se rejoignent au niveau du plan axial vertical médian du bac pour former une dite paroi de double fond, par rotation des volets autour de charnières à axes de rotation horizontaux fixées dur les parois 2c-1, 2c-2, solidaires des bords opposés 7a au niveau des dites parois latérales opposées longitudinales 2c-1 et 2c-2. D'autre part, les volets sont équipés de flotteurs. Les volets flottants sont ici fixés sur les parois du bac et pivotent vers le milieu mais ils pourraient également être fixés sur un axe au milieu du bac et pivoter de chaque côté vers les parois.

Ainsi, en l'absence de fluide dans le bac, figure 9B, les volets sont en position basse d'ouverture, les bords 7b en contact contre les parois respectives 2c-1, 2c-2, et lorsque l'on remplit le bac de fluide jusqu'à un niveau situé au-dessus des volets, les volets se ferment en position haute. Les deux volets en position haute de fermeture de la double paroi de fond, figure 9A, délimitent avec la paroi de fond 2b, un compartiment 8 servant de piège à produits coulés non flottants.

Ce compartiment piège 8 permet de confiner les produits coulés d'un premier traitement et ainsi d'éviter que leur emballage et/ou leur contenu ne se mélangent avec les produits à traiter lors d'un traitement subséquent d'un deuxième lot de produits. Il permet aussi de stocker les produits coulés provenant de différents lots dans le dit compartiment piège 8 que l'on évacue ultérieurement à l'aide d'une deuxième trappe 9 dans la paroi de fond 2b. Ceci permet d'éviter d'évacuer les produits coulés d'un lot précédent avant le traitement d'un lot suivant. A défaut de compartiment piège, il y un risque que les emballages coulés ne viennent à se séparer de leur contenu et ne se mélange dans le fluide avec les produits à traiter d'un lot suivant.

## Revendications

1. Dispositif de traitement thermique de produits (P) sous emballage disponibles en vrac comprenant une enceinte (1) destinée à être mise sous pression et à mettre en oeuvre un tel traitement thermique, **caractérisé en ce que** le dispositif comprend :
- un bac (2) destiné à contenir un fluide d'une densité telle que les produits (P) sous emballage flottent lorsqu'ils y sont déversés en vrac, ce bac (2) présentant des moyens d'évacuation (21,22) des produits (P) en état de flottaison, ces moyens d'évacuation comprenant une trappe d'évacuation (22) apte à être immergée puis ouverte pour permettre l'évacuation d'une partie du fluide comportant les produits (P) en état de flottaison,
- des moyens de fourniture de fluide (23) pour remplir le bac (2) et réguler le niveau de fluide (N1, N2) dans le bac (2),
- des moyens d'immersion (3) agissant au niveau de la surface supérieure de fluide présent dans le bac (2) pour immerger les produits (P) en vrac dans le fluide,
- des moyens de récupération (4) des produits (P) au sein du fluide évacué du bac (2) lors de l'ouverture du compartiment d'évacuation (21),
- des moyens de réception (5,5a) du fluide déversé lors de l'ouverture du compartiment d'évacuation (21).

2. Dispositif selon la revendication 2, **caractérisé en ce que** le dit bac est amovible pouvant être introduit et sorti de la dite enceinte par au moins une porte (11,12) de la dite enceinte de préférence ledit bac étant apte à coopérer avec des moyens de déplacement tels que des rails et/ou glissières (2-1,1-1) sur lesquels il est posé.

3. Dispositif selon la revendication 1 ou 2, **caractérisé en ce que** le dit bac comporte, de préférence à une extrémité en vis-à-vis d'une porte (11) de l'enceinte, un compartiment supérieur d'évacuation (21) dont une paroi forme ou comprend une dite trappe d'évacuation (22) du compartiment d'évacuation (21), la dite trappe étant ouvrable, de préférence par pivotement ou coulissement, le dit compartiment d'évacuation étant disposé de telle sorte que le nombre de produit à l'intérieur dudit compartiment d'évacuation en vis-à-vis de la dite trappe soit en nombre inférieur au nombre de produit total contenu dans le bac.

4. Dispositif selon l'une des revendications 1 à 3, **caractérisé en ce que** le compartiment d'évacuation (21) constitue une structure (21a) en surplomb s'étendant sur un des côtés (2a-2) du bac (2).

5. Dispositif selon la revendication 4, **caractérisé en ce que** le dit compartiment d'évacuation (21) comprend un plancher (21a) s'étendant horizontalement au-dessus du plancher (2b) de l'enceinte, au-delà et à partir du sommet d'une paroi transversale verticale (2a-2) inférieure dudit bac, jusque vers la porte de sortie (12) de l'enceinte, la dite paroi transversale verticale (2a-2) inférieure du bac délimitant une partie inférieure du bac dessous et amont du dit compartiment d'évacuation.

6. Dispositif selon l'une des revendications 3 à 5, **caractérisé en ce que** la dite trappe (22) du compartiment d'évacuation (21) est placée au niveau d'une paroi transversale verticale dudit compartiment d'évacuation à une extrémité du bac (2) disposée en vis-à-vis d'une sortie de l'enceinte (1).

7. Dispositif selon l'une des revendications précédentes, **caractérisé en ce que** les moyens mécaniques d'immersion (3) comprennent une ou plusieurs plaques percées ou une ou des grilles aptes à être montées et descendues entre une position émergée et une position immergée, position immergée dans laquelle les produits (P), piégés sous la plaque ou la grille, peuvent être immergés, de préférence dessous la dite trappe.

8. Dispositif selon l'une des revendications précédentes, caractérisé en ce les moyens de fourniture (6) de fluide comprennent des entrées de fluide situées du côté opposé au compartiment d'évacuation (21) et injectant du fluide pour diriger les produits en direction du compartiment d'évacuation (21).

9. Dispositif selon l'une des revendications précédentes, **caractérisé en ce que** les moyens de récupération (4) des produits (P) au sein du fluide évacué du bac (2) lors de l'ouverture de la dite trappe comprennent une rampe ou goulotte perforée inclinée, de préférence perforée, s'étendant à l'extérieur du dit bac depuis juste dessous la dite trappe et sont associés à des moyens de déshumidification ou de séchage (23) des produits évacues sur la dite rampe ou goulotte .

10. Dispositif selon l'une des revendications précédentes, **caractérisé en ce qu'**il comprend-des moyens de régulation de l'ouverture de la dite trappe (22).

11. Dispositif selon l'une des revendications précédentes, **caractérisé en ce que** ledit bac comprend des moyens formant une deuxième paroi de fond (7) ouvrable disposée au-dessus de la première paroi de fond (2b), la dite deuxième paroi de fond étant apte à adopter:
- une position de fermeture empêchant que des produits coulés ou des parties de produits coulés d'un lot précédent reposant sur la première paroi de fond ne se mélangent avec les produits flottants d'un lot suivant, à traiter thermiquement, immergés dans le fluide au-dessus de la deuxième paroi de fond, les produits non flottants dudit lot suivant étant déposés sur ladite deuxième paroi de fond en position de fermeture, et
- une position d'ouverture permettant de laisser tomber sur la dite première paroi de fond, les produits non flottants reposant précédemment sur ladite deuxième paroi de fond.

12. Dispositif selon la revendication 11 **caractérisé en ce que** lesdits moyens formant la deuxième paroi de fond (7) comprennent deux volets pivotants montés articulés sur les parois latérales longitudinales opposées (2c-1,2c-2) dudit bac au-dessus de ladite première paroi de fond, celle-ci comprenant une deuxième trappe d'évacuation (9), les dits volets étant aptes à flotter de sorte que :
- lorsque le niveau de fluide est situé au-dessous des bords (7a) des volets équipés de charnières fixées sur les dites parois longitudinales, les dits volets sont en dite position d'ouverture, et
- lorsque le niveau de fluide est situé au-dessus des bords (7a) des volets équipés de charnières fixées sur les dites parois longitudinales, les dits volets sont en dite position de fermeture.

13. Procédé de traitement thermique de produits (P) sous emballage disponibles en vrac mettant en oeuvre une enceinte (1) destinée à être mise sous pression, de préférence à l'aide d'un dispositif selon l'une des revendications 1 à 12 **caractérisé en ce qu'**il comprend les étapes suivantes consistant à :
- remplir un bac (2) placé dans l'enceinte (1) avec un fluide
- remplir le bac avec un lot de produits (P) sous emballage en vrac , ledit fluide étant d'une densité telle que les produits (P) sous emballage flottent lorsqu'ils y sont déversés dans le dit bac , le bac (2) présentant des moyens d'évacuation (21) des produits (P) en état de flottaison comprenant une trappe (22) apte à être immergée,
- immerger les produits (P) mécaniquement,
- clore l'enceinte (1),
- procéder au traitement thermique au sein de l'enceinte (1) par chauffage de l'eau au sein dudit bac et mise en pression de l'intérieur de l'enceinte,
- laisser remonter en flottaison les produits (P),
- ouvrir la trappe (22) d'évacuation des produits (P) en état de flottaison,
- alimenter le dit bac en fluide et faire circuler le fluide et/ou les produits pour réguler l'évacuation du fluide et des produits (P) au niveau de la trappe,
- récupérer les produits (P) au sein du fluide évacué à l'extérieur du bac (2),
- récupérer le fluide déversé.

14. Procédé selon la revendication 13 **caractérisé en ce qu'**il comprend les étapes suivantes consistant à :
- remplir de fluide un dit bac (2) présentant un compartiment d'évacuation (21) des produits (P) en état de flottaison comprenant une trappe (22) apte à être immergée, jusqu'à un premier niveau (N1) situé dessous la dite trappe,
- déverser les produits (P) en vrac au sein du bac (2),
- immerger les produits (P) mécaniquement,
- clore l'enceinte (1),
- procéder au traitement thermique au sein de l'enceinte (1),
- ajouter du fluide au sein du bac jusqu'à un second niveau (N2) permettant l'immersion du compartiment d'évacuation (21) et de la trappe (22),
-- laisser remonter en flottaison les produits (P),
- ouvrir la trappe (22) du compartiment d'évacuation (21) des produits (P) en état de flottaison,
- alimenter le dit bac en fluide et faire circuler le fluide et/ou les produits pour réguler l'évacuation du fluide et des produits (P) au niveau de la trappe, en régulant le niveau de fluide au sein du bac pendant l'évacuation des produits (P),
- récupérer les produits (P) au sein du fluide évacué du bac (2),
- récupérer le fluide déversé.

15. Procédé selon la revendication 13 ou 14 **caractérisé en ce que**
- les produits non flottants sont déposés sur des moyens formant une deuxième paroi de fond (7) en position de fermeture disposée au-dessus de la première paroi de fond (2b), la dite deuxième paroi de fond étant apte à adopter:
- une position de fermeture empêchant que des produits coulés ou des parties de produits coulés d'un lot précédent reposant sur la première paroi de fond ne se mélangent avec les produits flottants d'un lot suivant, à traiter thermiquement, immergés dans le fluide au-dessus de la deuxième paroi de fond, les produits non flottants dudit lot suivant étant déposés sur ladite deuxième paroi de fond en position de fermeture, et
- une position d'ouverture permettant de laisser tomber sur la dite première paroi de fond, les produits non flottants reposant précédemment sur ladite deuxième paroi de fond, et
- après que tous les produits flottants aient été évacués par la dite trappe d'évacuation (22), on ouvre la dite deuxième paroi de fond pour déposer sur la dite première paroi de fond, les produits non flottants reposant précédemment sur ladite deuxième paroi de fond.

## Patentansprüche

1. Vorrichtung zur Wärmebehandlung von ungeordnet vorhandenen, verpackten Produkten (P), umfassend einen Raum (1), der dazu bestimmt ist, unter Druck gesetzt zu werden und eine solche Wärmebehandlung durchzuführen, **dadurch gekennzeichnet, dass** die Vorrichtung umfasst:
- eine Wanne (2), die dazu bestimmt ist, ein Fluid mit einer Dichte zu enthalten, die derart ist, dass die verpackten Produkte (P) schwimmen, wenn sie ungeordnet dort hinein geschüttet werden, wobei diese Wanne (2) Mittel zum Abführen (21, 22) der Produkte (P) im Schwimmzustand aufweist, wobei diese Abführmittel eine Abführklappe (22) umfassen, die geeignet ist, eingetaucht, dann geöffnet zu werden, um das Abführen eines Teils des die Produkte (P) im Schwimmzustand enthaltenden Fluids zu ermöglichen,
- Fluidlieferungsmittel (6), um die Wanne (2) zu füllen und den Fluidpegel (N1, N2) in der Wanne (2) zu regulieren,
- Eintauchmittel (3), die in Höhe der Oberfläche von in der Wanne (2) vorhandenem Fluid wirken, um die in dem Fluid ungeordneten Produkte (P) einzutauchen,
- Mittel zum Auffangen (4) der Produkte (P) in dem beim Öffnen des Abführabteils (21) aus der Wanne (2) abgeführten Fluid,
- Mittel zur Aufnahme (5, 5a) des beim Öffnen des Abführabteils (21) abgelassenen Fluids.

2. Vorrichtung nach Anspruch 1, **dadurch gekennzeichnet, dass** die Wanne lösbar ist, wobei sie über wenigstens eine Tür (11, 12) des Raums eingeführt und aus dem Raum herausgenommen werden kann, wobei die Wanne vorzugsweise geeignet ist, mit Bewegungsmitteln, wie Schienen und/oder Gleitschienen (2-1, 1-1), auf die sie aufgesetzt ist, zusammenzuwirken.

3. Vorrichtung nach Anspruch 1 oder 2, **dadurch gekennzeichnet, dass** die Wanne vorzugsweise an einem Ende gegenüber einer Tür (11) des Raums ein oberes Abführabteil (21) umfasst, von dem eine Wand eine Abführklappe (22) des Abführabteils (21) bildet oder umfasst, wobei die Klappe geöffnet werden kann, vorzugsweise durch Verschwenken oder Schieben, wobei das Abführabteil derart angeordnet ist, dass die Produktanzahl innerhalb des Abführabteils gegenüber der Klappe geringer ist als die in der Wanne enthaltene Produktgesamtanzahl.

4. Vorrichtung nach einem der Ansprüche 1 bis 3, **dadurch gekennzeichnet, dass** das Abführabteils (21) eine überhängende Struktur (21a), die sich auf einer der Seiten (2a-2) der Wanne (2) erstreckt, bildet.

5. Vorrichtung nach Anspruch 4, **dadurch gekennzeichnet, dass** das Abführabteil (21) einen Boden (21a) umfasst, der sich oberhalb des Bodens (2b) der Wanne, jenseits des obersten Punktes einer unteren vertikalen Querwand (2a-2) der Wanne und hiervon ausgehend, bis zu der Austrittstür (12) des Raums horizontal erstreckt, wobei die untere vertikale Querwand (2a-2) der Wanne einen unteren Teil der Wanne unter und vor dem Abführabteil begrenzt.

6. Vorrichtung nach einem der Ansprüche 3 bis 5, **dadurch gekennzeichnet, dass** die Klappe (22) des Abführabteils (21) im Bereich einer vertikalen Querwand des Abführabteils an einem Ende der Wanne (2), das gegenüber einem Ausgang des Raums (1) angeordnet ist, angebracht ist.

7. Vorrichtung nach einem der vorhergehenden Ansprüche, **dadurch gekennzeichnet, dass** die mechanischen Eintauchmittel (3) eine oder mehrere durchbohrte Platten oder ein oder mehrere Gitter umfassen, die geeignet sind, zwischen einer aufgetauchten Position und einer eingetauchten Position herauf und herunter bewegt zu werden, eingetauchten Position, in der die unter der Platte oder dem Gitter gefangenen Produkte (P) vorzugsweise unter die Klappe eingetaucht werden können.

8. Vorrichtung nach einem der vorhergehenden Ansprüche, **dadurch gekennzeichnet, dass** die Fluidlieferungsmittel (6) Fluideinlässe umfassen, die auf der von dem Abführabteil (21) abgewandten Seite gelegen sind und Fluid einspritzen, um die Produkte in Richtung des Abführabteils (21) zu leiten.

9. Vorrichtung nach einem der vorhergehenden Ansprüche, **dadurch gekennzeichnet, dass** die Mittel zum Auffangen (4) der Produkte (P) in dem beim Öffnen der Klappe aus der Wanne (2) abgeführten Fluid eine gelochte, geneigte, vorzugsweise gelochte Rampe oder Rinne umfassen, die sich außerhalb der Wanne genau von unter der Klappe aus erstreckt, und Mitteln zum Entfeuchten oder Trocknen (23) der auf der Rampe oder Rinne ausgetragenen Produkte zugeordnet sind.

10. Vorrichtung nach einem der vorhergehenden Ansprüche, **dadurch gekennzeichnet, dass** sie Mittel zum Regulieren des Öffnens der Klappe (22) umfasst.

11. Vorrichtung nach einem der vorhergehenden Ansprüche, **dadurch gekennzeichnet, dass** die Wanne Mittel umfasst, die eine oberhalb der ersten Bodenwand (2b) angeordnete zweite offenbare Bodenwand (7) bilden, wobei die zweite Bodenwand geeignet ist, einzunehmen:
- eine Schließposition, die verhindert, dass ausgelaufene Produkte oder Teile von ausgelaufenen Produkten einer vorhergehenden Charge, welche auf der ersten Bodenwand aufliegen, sich mit den schwimmenden Produkten einer folgenden Charge, welche thermisch zu behandeln sind, oberhalb der zweiten Bodenwand in das Fluid eingetaucht sind, vermischen, wobei die nicht schwimmenden Produkte der folgenden Charge auf der zweiten Bodenwand in Schließposition abgesetzt sind, und
- eine Öffnungsposition, die ermöglicht, die nicht schwimmenden Produkte, die zuvor auf der zweiten Bodenwand auflagen, auf die erste Bodenwand fallen zu lassen.

12. Vorrichtung nach Anspruch 11, **dadurch gekennzeichnet, dass** die die zweite Bodenwand (7) bildenden Mittel zwei Schwenkklappen umfassen, die an den gegenüberliegenden Längsseitenwänden (2c-1, 2c-2) der Wanne oberhalb der ersten Bodenwand gelenkig angebracht sind, wobei diese eine zweite Abführklappe (9) umfasst, wobei die Klappen geeignet sind, hin- und herzugehen, so dass:
- wenn der Fluidpegel unterhalb der Ränder (7a) der Klappen, die mit an den Längswänden befestigten Scharnieren ausgestattet sind, liegt, die Klappen sich in der Öffnungsposition befinden, und
- wenn der Fluidpegel oberhalb der Ränder (7a) der Klappen, die mit an den Längswänden befestigten Scharnieren ausgestattet sind, liegt, die Klappen sich in der Schließposition befinden.

13. Verfahren zur Wärmebehandlung von ungeordnet vorhandenen, verpackten Produkten (P), das einen Raum (1) verwendet, der dazu bestimmt ist, unter Druck gesetzt zu werden, vorzugsweise mit Hilfe einer Vorrichtung nach einem der Ansprüche 1 bis 12, **dadurch gekennzeichnet, dass** es die folgenden Schritte umfasst, die darin bestehen:
- eine in dem Raum (1) angeordnete Wanne (2) mit einem Fluid zu füllen,
- die Wanne mit einer Charge von ungeordneten, verpackten Produkten (P) zu füllen, wobei das Fluid eine derartige Dichte aufweist, dass die verpackten Produkte (P) schwimmen, wenn sie dort hinein in die Wanne geschüttet werden, wobei die Wanne (2) Mittel zum Abführen (21) der Produkte (P) im Schwimmzustand aufweist, die eine Klappe (22) umfassen, welche geeignet ist, eingetaucht zu werden,
- die Produkte (P) mechanisch einzutauchen,
- den Raum (1) zu schließen,
- die Wärmebehandlung innerhalb des Raums (1) durch Erhitzen des Wassers in der Wanne und Unterdrucksetzen des Inneren des Raums zu vollziehen,
- die Produkte (P) auftreiben zu lassen,
- die Klappe (22) zum Abführen der Produkte (P) im Schwimmzustand zu öffnen,
- die Wanne mit Fluid zu beaufschlagen und das Fluid und/oder die Produkte umlaufen zu lassen, um das Abführen des Fluids und der Produkte (P) im Bereich der Klappe zu regulieren,
- die Produkte (P) in dem abgeführten Fluid außerhalb der Wanne (2) aufzufangen,
- das abgelassene Fluid aufzufangen.

14. Verfahren nach Anspruch 13, **dadurch gekennzeichnet, dass** es die folgenden Schritte umfasst, die darin bestehen:
- eine Wanne (2), die ein Abteil zum Abführen (21) der Produkte (P) im Schwimmzustand aufweist, das eine Klappe (22) umfasst, die geeignet ist, eingetaucht zu werden, bis zu einer unterhalb der Klappe gelegenen ersten Höhe (N1) mit Fluid zu füllen,
- die Produkte (P) ungeordnet in die Wanne (2) zu schütten,
- die Produkte (P) mechanisch einzutauchen,
- den Raum (1) zu schließen,
- die Wärmebehandlung innerhalb des Raums (1) zu vollziehen,
- in die Wanne Fluid bis zu einer zweiten Höhe (N2) zuzugeben, die das Eintauchen des Abführabteils (21) und der Klappe (22) ermöglicht,
- die Produkte (P) auftreiben zu lassen,
- die Klappe (22) des Abteils zum Abführen (21) der Produkte (P) im Schwimmzustand zu öffnen,
- die Wanne mit Fluid zu beaufschlagen und das Fluid und/oder die Produkte umlaufen zu lassen, um das Abführen des Fluids und der Produkte (P) im Bereich der Klappe durch Regulieren des Fluidpegels innerhalb der Wanne während des Abführens der Produkte (P) zu regulieren,
- die Produkte (P) in dem aus der Wanne (2) abgeführten Fluid aufzufangen,
- das abgelassene Fluid aufzufangen.

15. Verfahren nach Anspruch 13 oder 14, **dadurch gekennzeichnet, dass**
- die nicht schwimmenden Produkte auf Mitteln abgesetzt werden, die eine oberhalb der ersten Bodenwand (2b) angeordnete zweite Bodenwand (7) in Schließposition bilden, wobei die zweite Bodenwand geeignet ist, einzunehmen:
- eine Schließposition, die verhindert, dass ausgelaufene Produkte oder Teile von ausgelaufenen Produkten einer vorhergehenden Charge, welche auf der ersten Bodenwand aufliegen, sich mit den schwimmenden Produkten einer folgenden Charge, welche thermisch zu behandeln sind, oberhalb der zweiten Bodenwand in das Fluid eingetaucht sind, vermischen, wobei die nicht schwimmenden Produkte der folgenden Charge auf der zweiten Bodenwand in Schließposition abgesetzt sind, und
- eine Öffnungsposition, die ermöglicht, die nicht schwimmenden Produkte, die zuvor auf der zweiten Bodenwand auflagen, auf die erste Bodenwand fallen zu lassen, und
- nachdem alle schwimmenden Produkte über die Abführklappe (22) abgeführt worden sind, die zweite Bodenwand geöffnet wird, um die nicht schwimmenden Produkte, die zuvor auf der zweiten Bodenwand auflagen, auf der ersten Bodenwand abzusetzen.

## Claims

1. A heat-treatment device for heat treating packaged products (P) that are available in bulk, said device comprising a chamber (1) for putting under pressure and for performing such heat treatment, said device being **characterized in that** it further comprises:
· a tank (2) for containing a fluid of a density such that the packaged products (P) float when they are poured in bulk into the fluid, the tank (2) presenting evacuation means (21, 22) for evacuating floating products (P), the evacuation means including an evacuation hatch (22) that is suitable for being submerged and then opened so as to evacuate a portion of the fluid including the floating products (P);
· fluid supply means (6) for filling the tank (2) and for regulating the level (N1, N2) of fluid in the tank (2);
· submersion means (3) that act at the top surface of the fluid present in the tank (2) so as to submerge the bulk products (P) in the fluid;
· recovery means (4) for recovering the products (P) contained within the fluid that is evacuated from the tank (2) when opening the evacuation compartment (21); and
· reception means (5, 5a) for receiving the fluid that pours out when opening the evacuation compartment (21).

2. A device according to claim 2, **characterized in that** said tank is removable and can be inserted into and removed from said chamber via at least one door (11, 12) of said chamber, said tank preferably being suitable for co-operating with movement means such as rails and/or slideways (2-1, 1-1) on which it is placed.

3. A device according to claim 1 or claim 2, **characterized in that** said tank includes an upper evacuation compartment (21), preferably at an end facing a door (11) of the chamber, which upper evacuation compartment (21) has a wall that forms or includes a said evacuation hatch (22) of the evacuation compartment (21), said hatch being openable, preferably by pivoting or sliding, said evacuation compartment being arranged such that the quantity of product inside said evacuation compartment facing said hatch is a quantity that is less than that the total quantity of product contained in the tank.

4. A device according to any one of claims 1 to 3, **characterized in that** the evacuation compartment (21) constitutes an overhanging structure (21a) that extends from one of the ends (2a-2) of the tank (2).

5. A device according to claim 4, **characterized in that** said evacuation compartment (21) includes a floor (21a) that extends horizontally over the floor (2b) of the tank, beyond and away from the top of a lower vertical transverse wall (2a-2) of said tank towards the outlet door (12) of the chamber, said lower vertical transverse wall (2a-2) of the tank defining a bottom portion of the tank that lies below and upstream from said evacuation compartment.

6. A device according to any one of claims 3 to 5, **characterized in that** said hatch (22) of the evacuation compartment (21) is placed at a vertical transverse wall of said evacuation compartment at an end of the tank (2) that is arranged facing an outlet of the chamber (1).

7. A device according to any preceding claim, **characterized in that** the mechanical submersion means (3) comprise one or more perforated plates or one or more grids that are suitable for being raised and lowered between an out-of-fluid position and a submerged position, in which submerged position the products (P) trapped below the plate or the grind may be submerged, preferably below said hatch.

8. A device according to any preceding claim, **characterized in that** the fluid supply means (6) comprise fluid inlets that are situated at the end remote from the evacuation compartment (21) and that inject fluid for directing the products towards the evacuation compartment (21).

9. A device according to any preceding claim, **characterized in that** the recovery means (4) for recovering the products (P) contained within the fluid that is evacuated from the tank (2) when opening said hatch comprise a sloping ramp or chute that is preferably perforated and that extends outside said tank from just below said hatch, and they are associated with dehumidification or drying means (23) for dehumidifying or drying the products that are evacuated onto said ramp or chute.

10. A device according to any preceding claim, **characterized in that** it includes control means for controlling the opening of said hatch (22).

11. A device according to any preceding claim, **characterized in that** said tank includes means that form an openable second bottom wall (7) that is arranged above the first bottom wall (2b), said second bottom wall being suitable for adopting:
· a closed position that prevents sunken products or portions of sunken products of a preceding batch that lie on the first bottom wall from mixing with the floating products of a following batch to be heat treated that is submerged in the fluid above the second bottom wall, the non-floating products of said following batch being deposited on said second bottom wall in the closed position; and
· an open position that makes it possible to allow the non-floating products that previously lay on said second bottom wall to drop onto said first bottom wall.

12. A device according to claim 11, **characterized in that** said means that form the second bottom wall (7) comprise two pivotable flaps that are hinge mounted on the opposite longitudinal side walls (2c-1, 2c-2) of said tank, above said first bottom wall, said first bottom wall including a second evacuation hatch (9), said flaps being suitable for floating so that:
· when the fluid level is situated below the edges (7a) of the flaps fitted with hinges fastened to said longitudinal walls, said flaps are in said open position; and
· when the fluid level is situated above the edges (7a) of the flaps fitted with hinges fastened to said longitudinal walls, said flaps are in said closed position.

13. A heat-treatment method for heat treating packaged products (P) that are available in bulk, said method using a chamber (1) for putting the products under pressure, preferably with a device according to any one of claims 1 to 12, said method being **characterized in that** it comprises the following steps consisting in:
· filling a tank (2) placed in the chamber (1) with fluid;
· filling the tank with a batch of packaged products (P) in bulk, said fluid being of a density such that the packaged products (P) float when they are poured in bulk into said tank, the tank (1) presenting evacuation means (21) for evacuating floating products (P), said evacuation means including a hatch (22) that is suitable for being submerged;
· submerging the products (P) mechanically;
· closing the chamber (1);
· proceeding with heat treatment within the chamber (1) by heating the water within said tank and putting the inside of the chamber under pressure;
· allowing said products (P) to float upwards;
· opening the evacuation hatch (22) for evacuating the floating products (P);
· supplying said tank with fluid and causing the fluid and/or the products to flow so as to control the evacuation of the fluid and of the products (P) via the hatch;
· recovering said products (P) contained within the fluid that is evacuated outside the tank (2);
· recovering the poured out fluid.

14. A method according to claim 13, **characterized in that** it comprises the following steps consisting in:
· filling a said tank (2) that presents an evacuation compartment (21) for evacuating the floating products (P) and that includes a hatch (22) that is suitable for being submerged, with fluid up to a first level (N1) that is situated below said hatch;
· pouring the products (P) in bulk into the tank (2) ;
· submerging the products (P) mechanically;
· closing the chamber (1);
· proceeding with heat treatment within the chamber (1);
· adding fluid into the tank up to a second level (N2) enabling the evacuation compartment (21) and the hatch (22) to be submerged;
· allowing the products (P) to float upwards;
· opening the hatch (22) of the evacuation compartment (21) for evacuating the floating products (P);
· supplying said tank with said fluid and causing the fluid and/or the products to flow so as to control the evacuation of the fluid and of the products (P) via the hatch, by controlling the fluid level within the tank while the products are being evacuated;
· recovering the products (P) contained within the fluid that is evacuated from the tank (2);
· recovering the poured out fluid.

15. A method according to claim 13 or claim 14, **characterized in that**
· the non-floating products are deposited on means that form a second bottom wall (7) when in a closed position, which second bottom wall is arranged above the first bottom wall (2b), said second bottom wall being suitable for adopting:
· a closed position that prevents sunken products or portions of sunken products of a preceding batch that lie on the first bottom wall from mixing with the floating products of a following batch to be heat treated that is submerged in the fluid above the second bottom wall, the non-floating products of said following batch being deposited on said second bottom wall in the closed position; and
· an open position that makes it possible to allow the non-floating products that previously lay on said second bottom wall to drop onto said first bottom wall; and
· after all of the floating products have been evacuated via said evacuation hatch (22), said second bottom wall is opened so as to deposit, on said first bottom wall, the non-floating products that previously lay on said second bottom wall.
